# EUROPEAN PATENT SPECIFICATION

(11) **EP 2 724 630 B1**
(45) Date of publication and mention of the grant of the patent: **13.04.2016**
(21) Application number: 13189852.0
(22) Date of filing: 23.10.2013
(51) Int. Cl.: A24F 47/00

(54) **Atomizing device and electronic cigarette having same**
Zerstäubungsvorrichtung und elektronische Zigarette damit
Dispositif d'atomisation et cigarette électronique comportant celui-ci

(30) Priority: 23.10.2012 CN 201210408618
(43) Date of publication of application: 30.04.2014
(73) Proprietor: Shenzhen First Union Technology Co., Ltd., Shenzhen Guangdong (CN)
(72) Inventor: Li, Yonghai, Shenzhen Guangdong (CN); Xu, Zhongli, Shenzhen Guangdong (CN); Hong, Hepeng, Shenzhen Guangdong (CN); Feng, Ye, Shenzhen Guangdong (CN); He, Youlin, Shenzhen Guangdong (CN)
(74) Representative: Protector IP Consultants AS

(56) References cited:
- US-A1- 2011 303 231
- US-A1- 2012 260 927

## Description

### BACKGROUND

### 1. Technical Field

The present invention relates to electronic cigarettes, particularly to an atomizing device and an electronic cigarette having the atomizing device.

### 2. Description of Related Art

Atomizing devices are key components of electronic cigarettes. A typical atomizing device includes a glass fiber core and a heating coil winding around the glass fiber core. Two ends of the heating coil are electrically connected to a power source. In application, a tobacco solution absorbed in the glass fiber core is atomized by the heating coil to obtain a smoking effect. Such an atomizing device is known from US 2011/0303231 A.

During work, the entire heating coil generates heat. As the heating coil support supports the glass fiber core, the contact portion of heating coil support between the heating coil and the heating coil support may be scalded after a long time of high temperature produced by the entire heating coil, which may bring an abnormal taste and influence the atomizing effect.

In application, only the section of the heating coil winding around the glass fiber core atomizes the tobacco solution; then the remaining of the heating coil which does not wind around the glass fiber core would yield an energy surplus during work, thereby leads to an energy loss. In addition, the two ends of the heating coil electrically connected to the power source may generate a great welding contact resistance, which leads to another energy loss.

Therefore, what is needed is a new kind of atomizing device and electronic cigarette, which has little contact resistance and energy loss, and would not change the taste of the electronic cigarette.

### BRIEF DESCRIPTION OF THE DRAWINGS

Many aspects of the present atomizing device and electronic cigarette can be better understood with reference to the following drawings. The components in the drawings are not necessarily drawn to scale, the emphasis instead being placed upon clearly illustrating the principles of the present atomizing device and electronic cigarette. Moreover, in the drawings, like reference numerals designate corresponding parts throughout the several views.
FIG. 1 is a schematic assembled view of a glass fiber core, a heating coil and a heating coil support.
FIG. 2 is a schematic assembled view of a glass fiber core and a heating coil of FIG. 1.
FIG. 3 is a schematic view of a heating coil support.
FIG. 4 is a schematic view of an atomizing device in accordance with a first embodiment.
FIG. 5 is a schematic view of a screw sleeve.
FIG. 6 is a schematic view of an electrode ring.
FIG. 7 is a schematic view of an air pipe support in accordance with the first embodiment.
FIG. 8 is a schematic view of an atomizing device in accordance with a second embodiment.
FIG. 9 is a schematic view of an atomizing device in accordance with a third embodiment.
FIG. 10 is a schematic view of an electronic cigarette in accordance with a fourth embodiment.

### DETAILED DESCRIPTION

Embodiments of the present atomizing device and electronic cigarette will now be described in detail below and with references to the drawings.

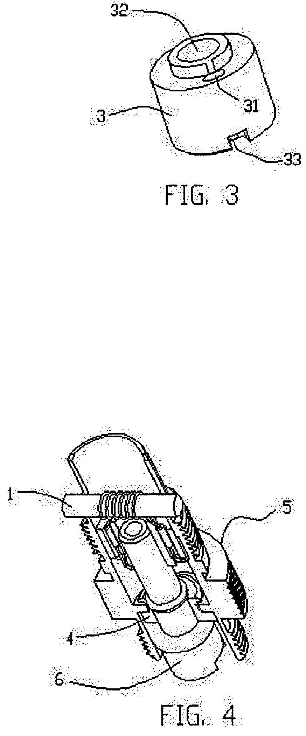

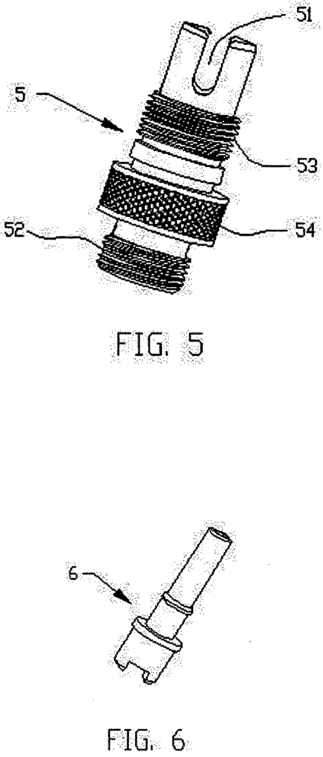

Referring to FIGS. 4 and 6, an atomizing device for an electronic cigarette includes a screw sleeve 5, an electrode ring 6 fixed in the screw sleeve 5 and an insulated ring 4 located between the electrode ring 6 and the screw sleeve 5. The screw sleeve 5 includes a glass fiber core 1, a heating coil 2 winding around the glass fiber core 1 and a heating coil support 3. Each of two ends 21 of the heating coil 2 is respectively connected to a silver coated bare electrical wire 22. The heating coil support 3 has a central through hole 32 and two side wall through holes 31 formed in the ring-shaped wall. In the present embodiment, the side wall through holes 31 and the central through 32 are parallel, but in other embodiments, they may not be parallel. In addition, the side wall through holes 31 are exposed to two ends of the heating coil support 3. Each of the bare electrical wires 22 extends respectively through the side wall through holes 31, then respectively in contact with the screw sleeve 5 and the electrode ring 6. As the ends 21 of the heating coil 2 are connected to the bare electrical wires 22, the ends 21 of the heating coil 2 do not in contact with the heating coil support 3. As known, silver has an excellent conductivity, and the silver coated bare electrical wires would generate little heat when electric current flows through the bare electrical wires, thus the heating coil support 3 would not be scalded.

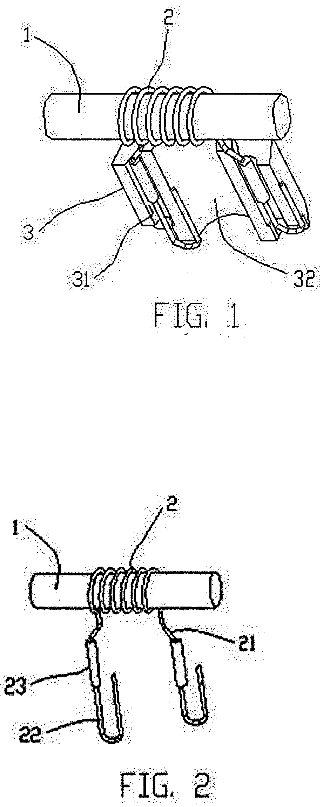

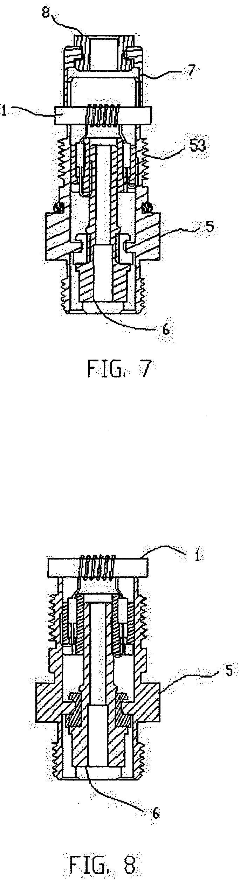

Referring to FIG. 1, as the bare electrical wires 22 extend through the side wall through holes 31, they are bended respectively at an the outer wall of the heating coil support 3 and an inner wall of the central through hole 32 (see FIG. 8), such that it is convenient to connect the electrode ring 6 to the atomizing device , and thus avoid welding.

In addition, in the present embodiment, the diameter of the bare electrical wire 22 is in a range between 0.2-0.4 mm. Although the greater the diameter of the bare electrical wire 22, the smaller the contact resistance, the bare electrical wire 22 in the present diameter range is more easily to insert into the side wall through hole 31.

Referring to FIG. 2, the ends 21 of the heating coil 2 are connected to the bare electrical wires 22 via riveting. Although there are many types of connection, for example, welding, it does not work very well due to the chemical reaction occurring in a high welding temperature. Via riveting, a contact 23 is formed between the end 21 and the bare electrical wire 22 as shown in FIG. 2.

Referring to FIGS. land 3, a cutout 33 is formed at an end of the heating coil support 3, such that the bare electrical wire 22 is bended at the outer wall of the heating coil support 3 through the cutout 33. Referring to FIG. 1, the cutout 33 allows one of the bended bare electrical wires 22 position at a different horizontal level from the other one of the bended bare electrical wires 22, thereby preventing the circuit from being cut out.

As known, the resistance changes inversely to the diameter of the conductor under the condition of a same material and a same resistivity, that is, the greater the diameter of the conductor, the smaller the resistance. Therefore, to yield enough energy to atomize the tobacco solution, a nichrome wire (Cr₂₀Ni₁₈) is selected to be the heating coil 2 in the present embodiment, and it is better that the diameter of the heating coil 2 is in a range between 0.09-0.16 mm. Specifically, when the diameter of the heating coil 2 is 0.12 mm, the volume of smoke is the greatest; when it is 0.09 mm or 0.16 mm, the volume of smoke is a little less than that when it is 0.12 mm.

Referring to FIGS 4 and 5, in order to more easily fix the glass fiber core 1, two arms extend from one end of the screw sleeve 5, then a receiving space 51 is formed between the two arms, then the glass fiber core 1 is fixed in the receiving space 51.

In addition, the diameter of the glass fiber core 1 is in a range from 1.5 to 2.5 mm in the present embodiment, and the number of coils of the heating coil 2 winding around the glass fiber core 1 is in a range between 3 to 7. According to experiment results, if the glass fiber core 1 is too thin, the tobacco solution absorbed in the glass fiber core 1 will be not enough, a smell of burnt will be generated due to repeatedly heating of the atomized tobacco solution; and if the glass fiber core 1 is too thick, the tobacco solution absorbed in the glass fiber core 1 will be redundancy, leading to the tobacco solution cannot be atomized sufficiently and the total volume of smoke will be not enough. Furthermore, if the glass fiber core 1 is too thick, the glass fiber core 1 would be stuck by an inner wall of the receiving space 51, and then the tobacco solution cannot reach the portion of the glass fiber core 1 where the heating coil 2 winds around. When the number of coils of the heating coil 2 is less than 3, the resistance of the heating coil 2 will become smaller with a constant voltage, the tobacco solution will be over-heated and generates a peculiar smell due to the over-heat produced by the heating section of the heating coil 2. When the number of coils of the heating coil 2 is more than 7, the total resistance will be greater due to the longer length of the heating coil, the amount of heat and smoke will be smaller with a constant voltage. Preferably, the diameter of the glass fiber core is 2.5 mm and the number of coils winded is 6, then the volume of smoke will be appropriate.

Referring to FIG. 5, a knurling 54 is formed at an outer wall of the screw sleeve 5. The knurling 54 can enhance a touch feeling when users hold the screw sleeve 5.

Referring to FIG. 5, one end of the screw sleeve 5 that fixing the glass fiber core 1 has an outer screw 53. Preferably, to facilitate connect and disconnect, an outer screw 52 and an outer screw 53 are respectively fixed at two ends of the screw sleeve 5. Accordingly, the outer screw 52 can be replaced by an inner screw.

Referring to FIG. 7, an air pipe support 7 is coupled upon one end of the screw sleeve 5, and then a sealing element 8 is fixed at the other end of the air pipe support 7. Due to the air pipe support7, the fiber glass core 1 can be fixed, and an airtight air funnel is formed upon the screw sleeve 5 to avoid the tobacco solution flowing towards the screw sleeve 5.

Referring to FIG. 8, an atomizing device in accordance with a second embodiment is provided. The difference of the atomizing device between the present embodiment and the first embodiment is that there is no receiving space 51 formed on the top end of the screw sleeve 5. Instead, a receiving space could be formed in the air pipe support 7.

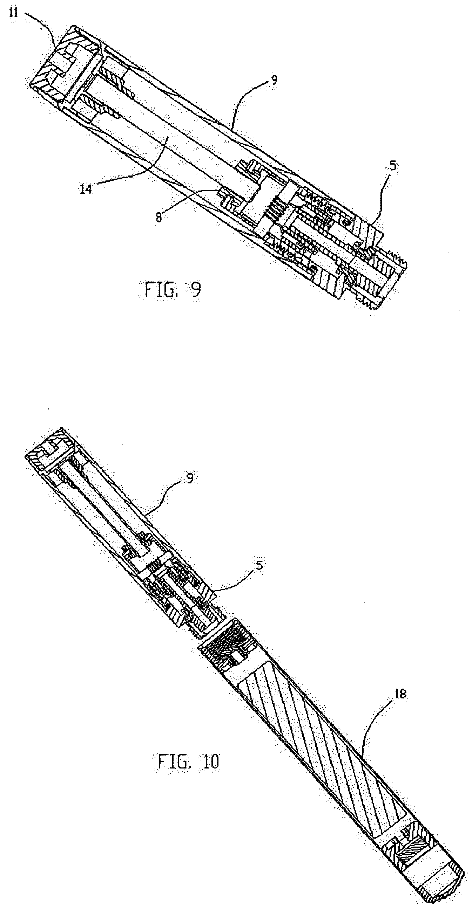

Referring to FIG. 9, an atomizing device in accordance with a third embodiment is provided. The atomizing device includes not only all the technical characteristics in the first embodiment but also an atomizing sleeve 9, a suction nozzle 11 arranged at one end of the atomizing sleeve 9 and an air pipe 14. The air pipe 14 is located in the atomizing sleeve 9, and is connected between the suction nozzle 11 and the atomizing device. Specifically, the air pipe support 7 is firstly arranged on the top end of the screw sleeve 5, then a sealing element 8 of the air pipe 14 is arranged on the top end of the air pipe support 7. Then the atomizing device is connected to the atomizing sleeve 9 via the outer screw 53, and the air pipe 14 is inserted into the sealing element 8. In the present embodiment, it is convenient to connect and disconnect between the atomizing device and the atomizing sleeve 9 to obtain an effect of directly adding tobacco solution to the atomizing sleeve 9 due to the outer screw 53. That is, after making use of the tobacco solution in the atomizing device, it is allowed to disconnect the atomizing device and add tobacco solution to the atomizing sleeve 9 to realize a recycled utilizing in the present embodiment.

Referring to FIG. 10, an electronic cigarette in accordance with a fourth embodiment is provided. The electronic cigarette includes not only all the technical characteristics in the fourth embodiment but also a battery assembly 18. The battery assembly 18 is configured to be electrically connected to the atomizing device.

## Claims

1. An atomizing device of an electronic cigarette, comprising:
a screw sleeve (5);
an electrode ring (6) fixed in the screw sleeve;
a glass fiber core (1) fixed in the screw sleeve (5);
a heating coil (2) winding around the glass fiber core (1)
a heating coil support (3) supporting the heating coil (2);
**characterized in that**
each of two ends of the heating coil (2) is respectively connected to a silver coated bare electrical wire (22), the heating coil support (3) comprises a central through hole (32) and two side wall through holes (31), the two bare electrical wires (22) respectively extend through the side wall through holes (31), then respectively in contact with the screw sleeve (5) and the electrode ring (6).

2. The atomizing device of claim 1, wherein the bare electrical wires extend through the side wall through holes, then are bended respectively at an outer wall of the heating coil support and an inner wall of the central through hole.

3. The atomizing device of claim 2, wherein a diameter of the bare electrical wire is in a range between 0.2 to 0.4 mm.

4. The atomizing device of claim 1, wherein the ends of the heating coil are connected to the bare electrical wires via riveting.

5. The atomizing device of claim 1, wherein the heating coil support comprises a cutout formed in an end of the heating coil support where the bare electrical wire is bended at.

6. The atomizing device of claim 1, wherein a diameter of the heating coil is in a range between 0.09 to 0.16 mm.

7. The atomizing device of claim 1, wherein a diameter of the glass fiber core is in a range between 1.5 to 2.5 mm; and a number of coils of the heating coil winding around the glass fiber core is in a range between 3 to 7.

8. The atomizing device of claim 1, wherein two arms extend from an end of the screw sleeve, then a receiving space is formed between the two arms, and the glass fiber core is fixed in the receiving space.

9. The atomizing device of claim 1, wherein a knurling is formed at an outer wall of the screw sleeve.

10. The atomizing device of claim 1, wherein one end of the screw sleeve comprises an outer screw.

11. The atomizing device of claim 1, wherein each of two ends of the screw sleeve comprises an outer screw.

12. An atomizing device, comprising
an atomizing sleeve;
a suction nozzle fixed at one end of the atomizing sleeve; and
an air pipe located in the atomizing sleeve;
wherein the other end of the atomizing sleeve comprises an atomizing device of claim 1, and the air pipe is connected between the suction nozzle and the atomizing device.

13. An electronic cigarette comprising a battery assembly and an atomizing device of claim 1, the battery assembly configured to be electrically connected to the atomizing device.

## Patentansprüche

1. Zerstäubervorrichtung einer elektronischen Zigarette, umfassend:
eine Schraubhülse (5);
einen in der Schraubhülse befestigten Elektrodenring (6);
einen in der Schraubhülse (5) befestigten Glasfaserkern (1);
eine Heizspule (2), welche sich um den Glasfaserkern (1) windet;
eine Heizspulenstütze (3), welche die Heizspule (2) stützt;
**dadurch gekennzeichnet, dass** jedes der beiden Enden der Heizspule (2) jeweils mit einem mit Silber beschichteten freiliegenden elektrischen Draht (22) verbunden ist, wobei die Heizspulenstütze (3) ein zentrales Durchgangsloch (32) und zwei Seitenwanddurchgangslöcher (31) umfasst, wobei die beiden freiliegenden elektrischen Drähte (22) sich jeweils durch die Seitenwanddurchgangslöcher (31) erstrecken und dann jeweils in Kontakt mit der Schraubhülse (5) und dem Elektrodenring (6) sind.

2. Zerstäubervorrichtung nach Anspruch 1, wobei sich die freiliegenden elektrischen Drähte durch die Seitenwanddurchgangslöcher erstrecken und dann jeweils an einer äußeren Wand der Heizspulenstütze und an einer inneren Wand des zentralen Durchgangslochs gebogen sind.

3. Zerstäubervorrichtung nach Anspruch 2, wobei ein Durchmesser des freiliegenden elektrischen Drahts im Bereich von 0,2 bis 0,4 mm ist.

4. Zerstäubervorrichtung nach Anspruch 1, wobei die Enden der Heizspule mit den freiliegenden elektrischen Drähten über eine Nietverbindung verbunden sind.

5. Zerstäubervorrichtung nach Anspruch 1, wobei die Heizspulenstütze einen in einem Ende der Heizspulenstütze gebildeten Durchbruch umfasst, wo der freiliegende elektrische Draht gebogen ist.

6. Zerstäubervorrichtung nach Anspruch 1, wobei ein Durchmesser der Heizspule in einem Bereich zwischen 0,09 und 0,16 mm ist.

7. Zerstäubervorrichtung nach Anspruch 1, wobei ein Durchmesser des Glasfaserkerns in einem Bereich von 1,5 bis 2,5 mm ist; und eine Anzahl von Spulen der Heizspulen, welche sich um den Glasfaserkern winden, im Bereich von 3 bis 7 ist.

8. Zerstäubervorrichtung nach Anspruch 1, wobei sich zwei Arme von einem Ende der Schraubhülse erstrecken, dann ein Aufnahmeraum zwischen den beiden Armen gebildet ist, und der Glasfaserkern im Aufnahmeraum fixiert ist.

9. Zerstäubervorrichtung nach Anspruch 1, wobei eine Rändel an einer äußeren Wand der Schraubhülse gebildet ist.

10. Zerstäubervorrichtung nach Anspruch 1, wobei ein Ende der Schraubhülse eine äußere Schraube umfasst.

11. Zerstäubervorrichtung nach Anspruch 1, wobei jedes der beiden Enden der Schraubhülse eine äußere Schraube umfasst.

12. Zerstäubervorrichtung, umfassend:
eine Zerstäuberhülse;
eine Saugdüse, welche an einem Ende der Zerstäuberhülse fixiert ist; und
ein in der Zerstäuberhülse angeordnetes Luftrohr;
wobei das eine Ende der Zerstäuberhülse eine Zerstäubervorrichtung nach Anspruch 1 umfasst und das Luftrohr zwischen der Saugdüse und der Zerstäubervorrichtung verbunden ist.

13. Elektronische Zigarette, umfassend einen Batterieaufbau und eine Zerstäubervorrichtung nach Anspruch 1, wobei der Batterieaufbau konfiguriert ist, um elektrisch mit der Zerstäubervorrichtung verbunden zu werden.

## Revendications

1. Dispositif d'atomisation d'une cigarette électronique, comprenant :
un manchon à vis (5) ;
un anneau d'électrode (6) fixé dans le manchon à vis ;
un noyau de fibre de verre (1) fixé dans le manchon à vis (5) ;
un enroulement de bobine de chauffage (2) autour du noyau de fibre de verre (1) ;
un support de bobine de chauffage (3) qui supporte la bobine de chauffage (2),
**caractérisé en ce que** chacune des deux extrémités de la bobine de chauffage (2) est respectivement connectée à un fil électrique nu gainé d'argent (22), le support de bobine de chauffage (3) comportant un trou traversant central (32) et deux trous traversants de paroi latérale (31), les deux fils électriques nus (22) s'étendant respectivement à travers les trous traversants de paroi latérale (31), ensuite respectivement en contact avec le manchon à vis (5) et l'anneau d'électrode (6).

2. Dispositif d'atomisation selon la revendication 1, dans lequel les fils électriques nus s'étendent à travers les trous traversants de paroi latérale, sont ensuite courbés respectivement à une paroi extérieure du support de bobine de chauffage et à une paroi intérieure du trou traversant central.

3. Dispositif d'atomisation selon la revendication 2, dans lequel un diamètre du fil électrique nu est compris dans la gamme de 0,2 mm à 0,4 mm.

4. Dispositif d'atomisation selon la revendication 1, dans lequel les extrémités de la bobine de chauffage sont connectées aux fils électriques nus par rivetage.

5. Dispositif d'atomisation selon la revendication 1, dans lequel le support de bobine de chauffage comporte une découpe formée dans une extrémité du support de bobine de chauffage à l'endroit où le fil électrique nu est courbé.

6. Dispositif d'atomisation selon la revendication 1, dans lequel un diamètre de la bobine de chauffage est compris dans la gamme de 0,09 mm à 0,16 mm.

7. Dispositif d'atomisation selon la revendication 1, dans lequel un diamètre du noyau de fibre de verre est compris dans la gamme de 1,5 mm à 2,5 mm ; et un certain nombre de spires de l'enroulement de bobine de chauffage autour du noyau de fibre de verre est compris dans la gamme de 3 à 7.

8. Dispositif d'atomisation selon la revendication 1, dans lequel deux bras s'étendent à partir d'une extrémité du manchon à vis, ensuite un espace de réception est formé entre les deux bras, et le noyau de fibre de verre est fixé dans l'espace de réception.

9. Dispositif d'atomisation selon la revendication 1, dans lequel un moletage est formé à une paroi extérieure du manchon à vis.

10. Dispositif d'atomisation selon la revendication 1, dans lequel une extrémité du manchon à vis comporte une vis extérieure.

11. Dispositif d'atomisation selon la revendication 1, dans lequel chacune des deux extrémités du manchon à vis comporte une vis extérieure.

12. Dispositif d'atomisation, comprenant :
un manchon d'atomisation ;
une buse d'aspiration fixée à une première extrémité du manchon d'atomisation ; et
un tuyau d'air situé dans le manchon d'atomisation,
dans lequel l'autre extrémité du manchon d'atomisation comprend un dispositif d'atomisation selon la revendication 1, et le tuyau d'air est connecté entre la buse d'aspiration et le dispositif d'atomisation.

13. Cigarette électronique comprenant un ensemble de batterie et un dispositif d'atomisation selon la revendication 1, l'ensemble de batterie étant configuré de manière à être électriquement connecté au dispositif d'atomisation.
